# EUROPEAN PATENT SPECIFICATION

(11) **EP 4 191 242 B1**
(45) Date of publication and mention of the grant of the patent: **17.06.2026**
(21) Application number: 21306691.3
(22) Date of filing: 02.12.2021
(51) Int. Cl.: G01N 33/24

(54) **DEVICE AND METHOD TO SIMULATE WETTING AND DRYING IN-SITU CONDITIONS ON A SAMPLE**
VORRICHTUNG UND VERFAHREN ZUR SIMULATION VON BENETZUNGS- UND TROCKNUNGSBEDINGUNGEN AN EINER PROBE IN SITU
DISPOSITIF ET PROCÉDÉ PERMETTANT DE SIMULER DES CONDITIONS DE MOUILLAGE ET DE SÉCHAGE IN-SITU SUR UN ÉCHANTILLON

(43) Date of publication of application: 07.06.2023
(73) Proprietor: Université Gustave Eiffel, 77420 Champs-sur-Marne (FR)
(72) Inventor: DAS, Geetanjali, 44400 Rezé (FR); RAZAKAMANANTSOA, Andry, 44360 Saint-Étienne-de-Montluc (FR); RAYSSAC, Erwann, 44830 Brains (FR)
(74) Representative: A.P.I. Conseil

(56) References cited:
- CN-A- 110 426 337
- OKEKE CHUKWUELOKA A U ED - PRIKRYL R: "Engineering behaviour of lime- and waste ceramic dust-stabilized expansive soil under continuous leaching", BULLETIN OF ENGINEERING GEOLOGY AND THE ENVIRONMENT, SPRINGER BERLIN HEIDELBERG, BERLIN/HEIDELBERG, vol. 79, no. 4, 4 December 2019 (2019-12-04), pages 2169 - 2185, XP037093555, ISSN: 1435-9529, [retrieved on 20191204], DOI: 10.1007/S10064-019-01648-2

## Description

### Field of the invention

The present invention relates to the field of the study of mechanical behavior of materials in response to environmental constraints. In particular, the invention relates to the field of test methods used to determine the evolution of specimens in regard to repeated wetting and drying cycles.

This invention provides a new device to simulate in-situ wetting and drying conditions and a method for simulating an in-field wetting and drying conditions, capable of being implemented by the device according to the invention. Similar devices are known from CN110426337A.

### Description of technical background

Continuous alteration of ingression and egression phenomenon occurs in the field soil structures that remain in contact with water bodies due to the fluctuations of water levels. Such a phenomenon induces continuous ingress and egress of liquids to and from a soil structure. The extent of ingress and egress of liquids is influenced by the surrounding temperature and relative humidity (RH). The occurrence of such phenomenon was shown to bring failure in soil structure and loss of chemical binders from treated soil structure.

Currently, in geotechnical engineering, the evaluation of the influence of wetting and drying cycles (W-D) is performed according to ASTM D559 (Standard, A. S. T. M. (2015). Standard Test Methods for Wetting and Drying Compacted Soil-Cement Mixtures. ASTM International, West Conshohocken, PA). This method is developed to determine the level of resistance of compacted soil treated by cement (or other binders) against field weathering induced by wetting and drying cycles. The process involves 5 hours of soaking the treated soil in water at room temperature followed by 43 hours of oven-drying at 71°C.

It should be note that during the soaking period, all specimens, irrespective of their configuration, are placed in a single cell containing the given water. Since all the specimens, regardless of their configuration, are placed in a single cell during the wetting and drying cycles according to the current process (Norm ASTM D559), the interaction between soil specimen and liquids cannot be considered. This can modify the physicochemical and microstructural properties of the soil. For instant, natural, or chemically treated soil are composed of minerals of exchangeable ions. Thus, the exchange of ions occurs during the wetting hours, when the soil is in contact with water. Based on the nature of the soil, this exchange mechanism varies (Mitchell, J. K., & Soga, K. (2005). Fundamentals of soil behavior (Vol. 3). New York: John Wiley & Sons). As a result, release and absorption of ions can occur, which in turn can modify the swelling and shrinkage in the respective soil. Thus, an inaccuracy in the measured swelling and shrinkage nature can result.

In addition, a constant wetting duration of 5 hours and a drying duration of 43 hours are imposed for all types of treated soils. This means the saturation level of the subjected material is ignored. The saturation level of treated soil is an important parameter to be considered for evaluating the quality of interactions between soil and chemical binders (Little, D. N. (1995). Stabilization of pavement subgrades and base courses with lime; and Le Runigo, B., Ferber, V., Cui, Y. J., Cuisinier, O., & Deneele, D. (2011). Performance of lime-treated silty soil under long-term hydraulic conditions. Engineering geology, 118(1-2), 20-28*).*

On another note, oven-drying a soil specimen at 71°C does not represent the actual drying temperature a soil experiences at the field.

Furthermore, soaking of the specimens is required to be made at room temperature, while drying is to be made in an oven at an elevated temperature of 71°C. Thus, a huge difference in temperature is implemented. Such an implementation does not represent the real field situation, where the occurrence of water ingression and egressions often occurs with a minimum temperature variation between the wetting and drying phases.

Besides, drying a soil at 71°C can trigger significant suction development, which in a way can led to misinterpretation (or overestimation) of the mechanical strength of a given treated soil.

Oven-drying of soil at elevated temperature (> 60°C) was shown to bring a modification of intrinsic soil structures *(*Sunil, B. M., & Deepa, A. V. (2016). Influence of drying temperature on three soils physical properties. Geotechnical and Geological Engineering, 34(3), 777-788*;* and Basma, A. A., Al-Homoud, A. S., & Al-Tabari, E. Y. (1994). Effects of methods of drying on the engineering behavior of clays. Applied Clay Science, 9(3), 151-164). For instant, Sunil and Deepa, reported that exposing bentonites with greater fine content to oven-drying removes the clay attached water and modifies the soil structure. This, in turn, modifies the soil geotechnical properties such as liquid limit, plastic limit, compaction parameters etc. Similarly, Basma et al., 1994 demonstrated an increase in swell potential, compressibility, and decreased unconfined compressive strength (UCS) of clay soil when exposed to oven-drying at high temperatures.

According to the current procedure (Norm ASTM D559), "specimens are required to be placed in the oven during inaccessible periods to the experimental location". Thus, the drying hours experienced by the specimen do not remain constant in every cycle. This can bring a discontinuity in the strength evolution and microstructural modifications between cycles, and hence the obtained analysis may not be accurate.

Consideration of RH is not made whereas RH is an important parameter that was shown to influence the extent of ingression and egression phenomenon in sample as soil for example. Moreover, cement- and lime-treatment of soil develops cementitious compounds such as Calcium-Silicate-Hydrates (C-S-H) within the soil pore-structure, which is responsible for the soil improvement (Das, G., Razakamanantsoa, A., Herrier, G., Saussaye, L., Lesueur, D., & Deneele, D. (2021). Evaluation of the long-term effect of lime treatment on a silty soil embankment after seven years of atmospheric exposure: Mechanical, physicochemical, and microstructural studies. Engineering Geology, 281, 105986*,* and Das, G., Razakamanantsoa, A., Herrier, G., & Deneele, D. (2021). Compressive strength and microstructure evolution of lime-treated silty soil subjected to kneading action. Transportation Geotechnics, 100568). However, the subjection of these treated soil to elevated temperature was shown to undergo destabilization of C-S-H, which is detrimental to the performances of treated soil (Gallucci, E., Zhang, X., & Scrivener, K. L. (2013). Effect of temperature on the microstructure of calcium silicate hydrate (CSH). Cement and Concrete Research, 53, 185-195*).*

Finally, it is mentioned that "the quality of the result produced by ASTM D559 test is dependent on the ability of the personnel performing it, and the suitability of the equipment and facilities used".

Document CN110426337 relates to geotechnical engineering tests and more precisely describes a rock and soil mass penetration deformation test device. The document is interested in measuring and quantifying axial and circumferential deformation of a sample under low stress condition and dry-wet cycle. To do that, the device comprises a vertical loading system which applies a force on the sample to be tested and an infiltration system.

Hence, a need exists for a solution to accurately monitor the behaviors of samples as compacted soil by submitting it to wetting, (soaking) and drying phenomena the closest as possible to those experienced in-situ conditions. In particular, the invention aims to evaluate the impact of recurrent ingress and egress of liquids in a given type of specimen by incorporating a situation closely representative of the in-situ conditions. More particularly the invention allows analysis of the chemical nature of liquids that remains in contact with the sample, simultaneously and continuously which is of the utmost interest in the field to get further understanding materials behavior to repeated wetting and drying cycles.

### Summary of the invention

The invention aims to overcome the disadvantages of the prior art.

In particular, the invention proposes a device to simulate wetting and drying in-situ conditions on at least two samples of a civil engineering material or a soil sample, according to claim 1.

Said device is particularly advantageous to accurately reproduce and simulate in-situ conditions to which the sample is exposed, and therefore allows an accurate evaluation of behavior of a civil engineering material or a soil sample.

In particular, said device make it possible to monitor and set numerous parameters, thereby allowing a better in-situ conditions. In that regard, an embodiment of the invention relates to the device as above wherein the predetermined parameters which are implemented and/or monitored are selected from: the predetermined volume, duration of wetting, duration of drying, number of wetting and drying cycles, temperature of the liquid, temperature within the separated space, pH, electric conductivity, input flows and/or output flows, of the liquid in the at least one separated space.

In another embodiment, the device according to the invention comprises several liquid collection tanks each configured to recover a predetermined volume of supernatant exiting from the at least one first separated space, at the end of different wetting. This is particularly advantageous as it allows to realize a given analysis of chemical/mineral species of the sample after each given wetting.

The device is configured to receive several separated spaces, thereby allowing, e.g., to test different predetermined parameters or analyze behavior. Accordingly, in an embodiment the device of the invention presents several separated spaces, each configured to accommodate each a sample. In a particular embodiment, the several separated spaces linked at their exit:
- all to one other liquid collection tank; or
- each to at least one liquid collection tank.

Also, the predetermined parameters are independently implemented and/or monitored in each of the separated spaces from the control panel.

In an embodiment, the device of the intention comprises:
- a pump;
- at least one sensor to monitor at least one predetermined parameter in each separated space individually, for instance the level of liquid;
- at least one valve controlling, independently from the other separated spaces, the input flow in each of the separated spaces, and/or
- at least one valve with controlled opening for controlling the output flow of the supernatant in one or several collection tanks.

The device according to invention comprises a climatic chamber configured to receive the separated spaces, which regulates the temperature and Relative Humidity around and/or in said separated space during the wetting and drying cycle. This can allow to control environmental conditions samples are submitted to and/or reproduce in-situ condition.

In an embodiment the device of the invention is configured to receive :
- a soil sample which is treated with a binder, the binder being mineral and/or organic, for instance lime, cement, fly ash, vegetable-based adhesive, resin, copolymers, or
- a civil engineering material which is soil, compacted soil, concrete, cement, bitumen or bituminous material, timber or any other material as steel.

As mentioned above and as it is further detailed in the description, the device is particularly suitable for simulating wetting and drying in-situ conditions on samples. Therefore, object, not part of the claimed invention, refers to the use of said device. Also, the invention relates to a method to simulate wetting and drying in-situ conditions on several samples of a civil engineering material or a soil sample, according to claim 6.

In a particular embodiment of said method the step of drying the sample is realized at a temperature inferior to 60°C, preferably between 15 and 27°C.

In another particular embodiment of the method, the duration of wetting and/or drying step, is defined as to reach a predetermined liquid saturation level in the at least one sample.

The device comprises several separated spaces which accommodate a sample. Accordingly, in the method, the device comprises several samples , and each sample undergoes several wetting and drying cycles. In said method the device comprises several samples each submitted to at least one different parameter of wetting and drying conditions. It can allow, e.g., the comparison of the behavior of one kind of sample, or even several different kinds of samples, to different parameter of wetting and drying conditions.

In an embodiment, the method comprises a step of performing at least one physico-chemical assay on at least one supernatant exiting from the at least one first separated space. This allows a more complete analysis of the behavior of the tested sample(s).

### Brief description of the drawings

The foregoing and other objects, features and advantages of the present invention will become more apparent from the following detailed description when taken in conjunction with the accompanying drawings in which:
FIG. 1 is a schematic view of a device according to an embodiment of the present invention.
FIG. 2 is a schematic view of a method according to an embodiment of the present invention.
FIG. 3 shows the evolution of the UCS in lime-treated soil samples subjected to W-D cycles as per ASTMD5559 procedure (AP) and method according to the invention set to simulate a W-D cycles expected during rainy season (RP, rainy protocol).
FIG. 4 shows the volume variation measured for lime-treated soil samples subjected to W-D cycles as per AP and RP.
FIG. 5 shows the water content variations in lime-treated soil samples subjected to W-D cycles as per AP and RP.
FIG 6a shows the suction and FIG 6b the pH measured in the initial and UCS subjected lime-treated soil samples during W-D cycles as per AP and RP.
FIG 7a shows Ca concentration and FIG 7b Electric Conductivity (EC) measured in the effluents collected from lime-treated soil samples subjected to the alternate cycles as per AP and RP.

### Detailed description

A description of example embodiments of the invention follows.

In the following, "**simulate an in-field wetting and drying conditions**" means artificial reproduction of real conditions linked in particular to wetting and drying phenomena to which material are (going to be) exposed.

As used herein, "**liquids**" refers to all kind of flowing substances to which a sample is susceptible to be exposed to in real conditions. It refers generally, but not only, to a liquid comprising at least water and which corresponds, is closed to, or reproduces at least one physicochemical property (e.g. the pH or the basicity), of the fluid(s) to which the sample(s) is(are) exposed in real conditions.

Except when otherwise stated, "fluid" or "solution" or "liquid" are used interchangeably. The term "**supernatant**" more specifically relates to the fluid, solution, liquid or effluent which exits from separated space(s) of the device, during or after the egression step. Said supernatant may comprise chemical substances originating from the tested sample and provide information on the behavior of the sample.

As used herein, a "**separated space**" means a physically delimited space as, for example, a box, a container or receptacle adapted to receive something inside. As used herein, said space is adapted to receive a sample of the material intended to be subjected to wetting and drying conditions. Consequently, two (or three or more) separated spaces can designates two (or three or more) physically distinct containers physically separated from each other. Alternatively, two (or three or more) separated spaces can designate contiguous spaces that separated by at least one common wall that delineate two (or here or more) distinct volumes which are not fluidically connected. According to the invention a separated space is configured to accommodate only one sample.

As used herein within the meaning of the invention, two compartments that are "**fluidically connected**" means that a fluid may flow through from the first to the second compartment by means of, e.g., a pipe, hose, a channel or duct. Fluid movement can be controlled by any convenient means in order to generate, slow or stop the flow between said compartments, by means of, e.g., a valve or a pump.

By "**a controlled manner** "within the meaning of the invention, an action performed by a device to control a flow. The controlled manner may be in the space or in the time. The controlled manner may be made manually or automatically. As explain above, the fluid movement can be controlled by any convenient means in order to generate, slow or stop the flow between said compartments, by means of, e.g., a valve or a pump.

As used herein "**coupled**" within the meaning of the invention, is meant connected, directly or indirectly, with one or more intermediate elements. Two elements may be coupled mechanically, electrically or linked by a communication channel.

The expression "**human-machine interface**" within the meaning of the invention, corresponds to any element allowing a human being to communicate with a particular computer and without this list being exhaustive, a keyboard and means allowing in response to the commands entered on the keyboard to perform displays and possibly to select using mouse or touchpad items displayed on the screen. Another exemplary embodiment is a touch screen making it possible to select directly on the screen the elements touched by the finger or an object and possibly with the possibility of displaying a virtual keyboard
As used herein "**processor**" within the meaning of the invention, is meant at least one hardware circuit configured to perform operations according to instructions contained in a code. The hardware circuit may be an integrated circuit. Examples of a processor include, but are not limited to, a central processing unit, a graphics processor, an application-specific integrated circuit (ASIC), and a programmable logic circuit.

By "**process**", "**calculate**", "**determine**", "**display**", "**extract**" "**compare**" or more broadly "**executable operation**" within the meaning of the invention an action performed by a device or a processor unless the context indicates otherwise. In this regard, operations refer to actions and / or processes of a data processing device, for example a computer system or an electronic computing device, which manipulates and transforms the data represented as physical (electronic) in computer system memories or other information storage, transmission or display devices. These operations can be based on applications or software.

"**UCS**" is the abbreviation for Unconfined Compressive Strength.

"**RH**" is the abbreviation for Relative Humidity.

"**W-D**" is the abbreviation for Wetting-Drying.

"**RP**" is the abbreviation for Rainy Protocol.

"**EC**" is the abbreviation for Electric Conductivity.

"**ICP-OES**" is the abbreviation for Inductively Coupled Plasma Optical Emission Spectrometry.

"**AP**" is the abbreviation for ASTM D559 Procedure (Norm ASTM D559 - 2015).

"**C-S-H**" is the abbreviation for Calcium-Silicate-Hydrates compounds.

As used herein, when a range is specified, the bounds are included.

As mentioned, using the current AP (ASTM D559 Standard) implies interactions between specimen which are soaked within the same compartment. Further, the modification (physicochemical and microstructural properties) of sample cannot be considered, the saturation level of the tested specimen is ignored. This procedure also implies 5 hours of soaking at room temperature followed by 43 hours of oven drying at 71°C which quite different of the real conditions leading to, as shown in the experimental section, modification of the intrinsic sample structures, discontinuity in the strength evolution, destabilization of C-S-H, which results in biased results about resistance of tested sample.

It has been discovered a new device which allows to incorporate a situation that closely represents the field conditions and submit samples of materials to such situation while monitoring their behavior This device is particularly adapted to study civil engineering materials as soils, compacted soils, soil treated with a binder, concretes, cements, bitumen or bituminous materials, timber, or any other material as (reinforcing or structural) steel, which behavior to wetting and drying cycles is questioned. It will be easily understood that a binder can be of any kind, either mineral and/or organic, for example said binder can be lime, cement, fly ash, vegetable-based adhesive, resin, copolymers. Accordingly, the device of the invention finds its applications in many types of industry, more particularly those which use geotechnology, concrete technology, steel technology and/or timber technology. Indeed, a device according to the invention enables an easy adaptation and control of the testing conditions (temperature, wetting, drying, and duration), of ingression and egression phenomenon, related to environmental and weathering conditions to be tested. In addition, thanks to the device according to the invention, different specimens (samples) (in regard to, e.g., their composition and/or their physicochemical properties) can be subjected to liquids of different chemistry. Furthermore, the device allows evaluating the physicochemical interactions between each specimen and liquids accurately and separately, with minimum disturbance to the specimens, without interaction or chemical exchange between said specimens. According to a **first aspect**, the invention relates to a **device 1 to simulate an in-field wetting and drying conditions.**

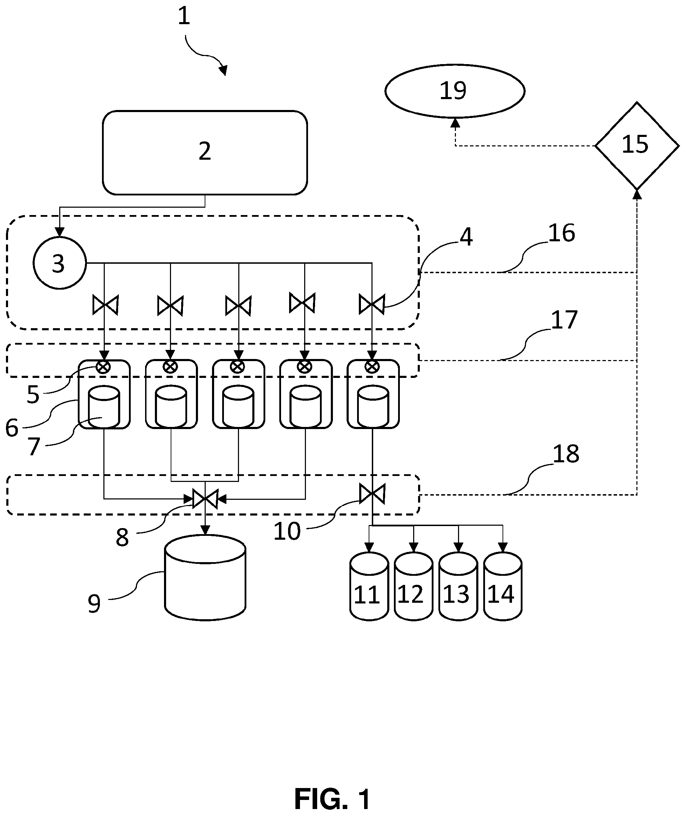

As shown in **figure 1**, said device 1 comprises a tank 2 adapted to comprise a predetermined volume of a liquid, several separated spaces 6, each adapted to accommodate a sample 7 and fluidically connected to the tank 2 in a controlled manner, at least one liquid collection tank 11 configured to recover fluid exiting from separated space 6, said liquid collection tank 11 and each separated space 6 being fluidically connected in a controlled manner, at least one control panel 15 configured to implement and/or monitor predetermined parameters simulating in-situ wetting and drying.

"**in situ** "within the meaning of the invention, represent conditions that are intended to be representative of the actual conditions and/or environmental conditions of the samples. The in-situ conditions can be in-situ soil conditions or in-situ civil engineering conditions, both represent the environmental conditions of the sample in its usual environment or in its usual use. The sample can be taken from a site and the device reproduces all the parameters of the site of origin. The liquid may correspond to all flowable substance to which the sample is susceptible to be exposed to in real conditions. In a particular embodiment, the liquid is an aqueous solution being selected from solutions as water, dematerialized water, sea water, wastewater, used water, acid rain, rain (or reconstituted solutions that mimic these solutions in their chemical composition or one or several physicochemical property). In addition, the device 1 may comprise several tanks 2 of solution each one comprising the same and/or different liquid.

The solution is preferably stored in **at least one tank 2** adapted to comprise a predetermined volume of a **liquid.** In order to reproduce real conditions and liquid needs; the tank 2 may be controlled in temperature, which is chosen to fit with in-field temperature, for example between 0°C and 50°C, preferably between 10°C and 40°C, and more preferably between 20°C and 25°C. The tank 2 of liquid may be placed at room temperature or at a predetermined temperature, allowing to reproduce the real condition. Preferably, the tank 2 is at room temperature.

In addition, the tank 2 may have a capacity of between 20L and 30L, preferably between 21L and 29L, preferably between 22L and 28L, preferably between 23L and 27L, preferably between 24L and 26L, more preferably a capacity of 23.120L or 25.432L or 27.744L.

The device according to the invention comprises several separated spaces 6, each adapted to accommodate a sample. As exposed above, the sample(s) may be a sample of a soil, compacted soil, concrete, cement, alkali-activated material(s), bitumen or bituminous material(s), timber, or any other material as (reinforcing or structural) steel (as e.g., steel of steel piles). Preferably, the sample(s) meet ASTM D559 standard requirements with respect to their size and form. Accordingly in a preferred embodiment the device is adapted to accommodate sample(s) of the size and/or form as specified by ASTM D559 standard.

The device is adaptable and transposable to many different types of technologies and studies. For example, in the geotechnical field, the device allows to reproduce and study the impact of ingression and egression phenomenon on the performances of soil or compacted soil structures; in building technology using timber: the device allows to reproduce and study the impact of moisture ingress and egress on the moisture gain, dimensional change, and structural integrity of mass timber panels; in concrete technology: the device allows to reproduce and study the chemical change in concrete and changes in concrete structures due to ingression of aggressive compounds such as sodium, magnesium, sulphate, chloride, carbonate etc...; in Steel technology: the device allows to reproduce and study the impact of chloride ingression in corrosion of steel structures.

As each sample is received in a separated space, the device according to the invention allows apply one condition to several samples or a specific condition to each (group of) sample.

Also, advantageously, the samples 7 come from identical technology and the device may serve to test different wetting and drying condition. In another advantageous embodiment, the samples 7 come from different technology and the device may serve to compare behavior of said samples submitted it to the same drying and wetting conditions. This saves time by processing several samples and/or conditions at once with minimal experimental variations.

Accordingly, the device comprises several separated spaces, preferably at least two separated spaces 6, more preferably at least three separated spaces 6 and even more preferably at least five separated spaces 6. A space may be a container, a box, a vessel, a receptacle, a volume separated from the other contiguous spaces by at least one wall.

Preferably, each space is separated physically from each other, in other words, there is no fluidic connection between two separate spaces. This makes it possible to avoid any interaction between the samples. This also makes it possible to save each sample from contamination and to adapt the treatment to each sample.

Each sample is placed separately in different spaces; thus, the physicochemical interactions between specimen and liquids and the microstructural modifications can be monitored accurately. In addition, as two specimens (samples) can be placed at one time hence, more results in a short period of time can be achieved with minimum labor.

In addition, each space may be closed. Moreover, each space may be at a controlled temperature, atmospheric pressure, and/or humidity. In particular each space may be at a specific controlled temperature, atmospheric pressure, and/or humidity.

Advantageously, the device comprises a climatic chamber. The climatic chamber may comprise all the separated spaces of the device. Alternatively, in an example which is not part of the present invention, the device as described above is incorporated in a climatic chamber. The climatic chamber can be an external, independent and movable box to regulate the temperature and relative humidity during wetting and drying durations. The volume of the chamber will be a total volume of all the separated spaces 6 including the spaces between each box. The climatic chamber will not be connected to control panel 15 but the sensor regulating the temperature and humidity will be connected. The parameters of the climatic chamber will be described below.

According to an embodiment of the invention the operating pressure is atmospheric pressure. According to an embodiment of the invention the relative humidity may be between 0 % and 100 %, preferably 100 %.

Advantageously, parameters are controlled to closely represents the field conditions.

In addition, the device may comprise pump 3, sensor to monitor at least one predetermined parameter, valve 4, 8, 10.

Sensor may be selected from a thermometer, pressure sensor, humidity sensor, pH meter, EC meter and/or level sensor. Preferably, each space may comprise at least one sensor.

The pump 3 may be configured to pump the solution from the tank 2 to each separated space 6, advantageously according to a predetermined liquid volume and filing velocity. Preferably, the pump is configured to pump the liquid from the tank 2 to the bottom of each separated space. Indeed, this makes it possible to ensure a laminar flow regime in order to reduce erosion or turbulent flow on the sample studied and therefore its possible degradation.

The valves 4, 8, 10 may be configured to let in and / or out the solution, fluids from samples, supernatants, and other collected fluids from sample, preferably without turbulence in regard to the sample. In addition, valves allow to fluidly connect the tank to the at least one separated space in a controlled manner. Preferably, each separated space comprises a valve 4 at their inlet. A valve may be a solenoid valve, directional control valve, pressure control valve, flow control valve, reed valve, blade connection valve, modular valve, cartridge valve, manual valve. The valves can control the input flow of fluid and output flow of fluid.

Preferably the device according to the invention comprises **a level sensor 5** for each separated space 6. The level sensor is configured to sense a predetermined level of the liquid. Advantageously, the level of the liquid may vary between spaces and therefore, from a sample to another. In addition, this allows to precisely know the wetting level of each sample, to detect the level of liquid in each space and to adapt the volume of liquid according to the sample, for example according to its porosity. A level sensor may be a level probe, a float, a camera, a laser sensor. Preferably, the solution penetrates the separated space from the top, thanks to the pump 3, and, when the level sensor detects that the desired volume is reached, feedback is set up by the control panel (which is detailed later). For example, it can be when the level sensor (a float) reaches the top of the separated space (the top being to the opposite position of the solution entrance).

The device according to the invention comprise **at least one liquid collection tank 11** configured to recover fluid from at least one first separate space 6 comprising a sample 7, Preferably without turbulence in regard to the sample. According to an embodiment of the present invention, the device may comprise several liquid collection tanks 11. For example, one for each sample or several for at least one first sample. The liquid collection tank 11 may be configured to recover fluid from only one sample 7. Fluid may correspond in this case to the supernatant. Preferably, when the device comprises several liquid collection tanks 11 12, 13, 14 for only one sample, each liquid collection tank collects fluid from the same sample corresponding to different cycles W-D. For example, in the embodiment with at least five separated spaces as illustrated in figure 1, a first liquid collection tank 11 is configured to collect fluid (i.e. supernatant) from one sample resulting from a first cycle W-D, a subsequent liquid collection tank 12 is configured to collect fluid from the same sample but from a subsequent cycle W-D, a further subsequent liquid collection tank 13 is configured to collect fluid from the same sample but at the another subsequent cycle W-D and so on. The present device consists of 5 boxes to receive specimens. In this implementation, the specimens used are identical, the chemical analysis of effluent obtained from only the 5th specimen was evaluated, considering it remained same for the remaining four identical specimens. The effluent from the first four specimens were collected in the same tank. However, the attached sensor can be multiplied if effluents are needed to be analyzed for each specimen given, they are not identical. In this aspect, the collection tank will also increase for individual chemical analysis. The interest is to investigate the chemical nature of the supernatant obtained after different cycles. This gives an insight of the leaching phenomenon occurring during the successive W-D cycles. Indeed, as the number of cycles increase, the successive ingression and egression episodes between the subjected sample and liquid influence the concentration of leached substances in the tested sample. In addition, other analyzes may be conducted on the effluent collected during the successive cycles as pH, EC, ICP, OES. The present invention simulates W-D cycles with conditions implemented close to field situation. Thus, the effect of the implementation on the chemical nature of the supernatant can be investigated. In another embodiment, the device comprises several liquid collection tanks 11 for only one sample of the first separated space for each W-D cycle, each fraction of supernatant so collected being dedicated for different physico-chemical analyses or assays. It will be appreciated that the invention is not limited to the number of liquid collection tank and its/their link(s) with said liquid collection tank, and several alternatives may be planned.

The device 1 according to the invention may comprise **at least one liquid collection tank 9.** According to another embodiment of the present invention, the device may comprise several liquid collection tanks 9.

The liquid collection tank 9 is configured to collect effluents from sample of at least one second separated space and preferably from several samples from different several second separated spaces. Advantageously, only one liquid collection tank 9 is configured to collect effluents from several samples from different several second separated spaces. More advantageously said only one solution collection tank is configured to collect fluids all through the wetting and drying procedure (that is for all the cycles of the process). Alternatively, several solution collection tanks 9 may be configured to collect each one fluid from one sample.

For example, when the device comprises five samples, supernatant from the first 4^{th} samples is collected in the solution tank 9, and effluent from the 5^{th} sample is gathered in the liquid collection tank 11 for, e.g., further analysis. Such configuration is particularly useful to save place, thereby limiting dimensions of the device.

The recovered solution in tank 9 may be reused or thrown out.

In case where there's no need to recover fluids or supernatants from the W-D cycles (e.g. when the WD simulation is concerned with effects on the sample such as erosion and not with the composition of the supernatant), then only one solution tank 9 is provided which gather all fluids from all separate spaces, and no liquid collection tank 11.

The device according to the invention may **comprise at least one control panel 15** configured to implement and/or monitor predetermined parameters.

Preferably, parameters are selected from: the order of the wetting and drying steps, number of wetting and drying cycles, temperature of the solution in the solution tank 2, temperature within the separated space, temperature of the solution in the fluid tank 11, temperature of the solution in the solution tank 9, relative humidity within the separated space, duration of wetting and duration of drying defined based on sample saturation level, pH, basicity, salt composition of the solution, inlet and/or outlet volumes of liquid of separated space, the rate of entry of the solution into the separate space, the rate of draining of the separate space.

Preferably, predetermined parameters are depending on sample and predetermined according to the sample. Also, predetermined parameters vary as a function of the W-D conditions reproducing the field situation(s) and environment(s) to be tested.

Advantageously, each parameter may be configured, applied, and/or monitored independently to each sample and/or separated space.

The parameter **order of the wetting and drying steps** may comprise which of wetting or drying step to start with. For example, either wetting first then drying or vice versa.

The parameter **number of wetting and drying cycles** may comprise the number of cycles W-D or the number of wetting for each sample or the number of drying for each sample. Preferably, the number of cycles is determined as a function of the testing requirement which can vary e.g. as a function of the type of sample which is subjected to WD cycles or to the field conditions that are tested/reproduced (as weather condition for example). As state above, a particular advantage of the device is that it requires a minimal handling of the samples, therefore basically not limiting the number of W-D cycles that could be applied. For instance, the number of cycles W-D is at least 1, preferably at least 2, preferably at least 3, preferably at least 4, preferably at least 5, preferably at least 6, preferably at least 7, preferably at least 8, preferably at least 9, preferably at least 10, preferably at least 11, preferably at least 12, preferably at least 13, preferably at least 14, preferably at least 15, preferably at least 16, preferably at least 17, preferably at least 18, preferably at least 19, preferably at least 20, preferably at least 30, preferably at least 40, preferably at least 50, preferably at least 60, preferably at least 70, preferably at least 80, preferably at least 90, preferably at least 100, preferably at least 110, preferably at least 120, preferably at least 130, preferably at least 140, preferably at least 150, preferably at least 160, preferably at least 170, preferably at least 180, preferably at least 190, preferably at least 200, preferably at least 300, preferably at least 400, preferably at least 500, preferably at least 600, preferably at least 700, preferably at least 800, preferably at least 900, and more preferably at least 1000. Preferably, the parameters number of wetting and drying cycles is configured according to the cycle number after which one wish to collect the supernatant exiting the separate space.

The parameters duration of wetting or duration of drying can be set independently based on the predetermined saturation level of the given sample. Preferably, the durations may be configured with the switch board for each sample. In addition, the duration of cycles may depend on the saturation level of the sample which is desired which may depend on the type of sample subjected to W-D cycles and/or on the conditions which are tested. The durations may be, each independently, for example between 1 min and 10000 min, preferably between 2 min and 9500min, preferably between 3 min and 9000min, preferably between 4 min and 8000min, preferably between 5 min and 7500min, preferably between 6 min and 7000min, preferably between 7 min and 6500min, preferably between 8 min and 6000min, preferably between 9min and 5500min, preferably between 10 min and 5000min, preferably between 11 min and 4500min, preferably between 12 min and 4000min, preferably between 13 min and 3500min, preferably between 14 min and 3000min, preferably between 15 min and 2500min, preferably between 16 min and 2000min, preferably between 17 min and 1500min, preferably between 18 min and 1000min, preferably between 19 min and 900min, preferably between 20 min and 800min, preferably between 21 min and 750min, preferably between 22 min and 700min, preferably between 23 min and 600min, preferably between 24 min and 500min, preferably between 25 min and 450min, preferably between 26 min and 400min, preferably between 27 min and 350min, preferably between 28 min and 300min, preferably between 29 min and 250min, preferably between 30 min and 200min, preferably between 31 min and 190min, preferably between 32 min and 180min, preferably between 33 min and 170min, preferably between 34 min and 160min, preferably between 35 min and 150min, preferably between 36 min and 140min, preferably between 37 min and 130min, preferably between 38 min and 120min, preferably between 39 min and 110min, preferably between 40 min and 100min, preferably between 41 min and 90min, preferably between 42 min and 80min, preferably between 43 min and 70min, preferably between 44 min and 60min, and more preferably between 45 min and 50min.

Other parameters affecting wetting or drying cycle are for example solution inlet and/or outlet volume of liquid of separated space the rate of entry of the liquid into the separate space, , the rate of draining of the solution out of the separate space.

The parameters **Temperatures** may comprise the temperature within the separated space, in the climatic chamber, temperature of the solution tank 2 solution, temperature of the liquid collection tank 11, of the solution collection tank 9. Advantageously, each temperature may be different according to the cycles, the number of cycles, the duration of cycles. For example, the temperature may be different between wetting and drying steps, between the first cycle W-D and the second cycle W-D, between the first sample and the second separates space etc. Preferably, the temperature during wetting or drying is ambient temperature comprised between 15 °C and 27 °C, preferably between 20-25 °C.

Preferably, the temperature inside the climatic chamber, when present, is comprised between 10 °C and 60 °C, and can be controlled to remain steady and inferior to 55 °C, 50 °C, 45°C, 40 °C, 35°C, 25 °C or even 20 °C.

Advantageously the **rate of entry of the solution into the separate space** and/or **the rate of draining of the solution out of the separate space** (i.e. the speeds) may also be predetermined. The control panel may be configured to define, monitoring and/or control the speed at which the solution enters the separated space, preferably in each separated space. The control panel may be configured to define the speed at which the solution exits the separated space, preferably in each separated space, once the soaking step ended, preferably in each separated space. The flow rate is controlled by the panel and/or by the valves. The flow of fluid is controlled so that the entry and exit of the fluid does not damage the sample such as by erosion. The fluid input and output are located so that the fluid emission is not in direct contact with the sample.

Advantageously, the control panel may be configured to be coupled with the device. According to an embodiment of the present invention, the control panel may be configured to control the pump 3 and the valve 4 via a first connection 16. The velocity of the pump, the pressure and the volume of the liquid may be also predetermined and configured from the control panel via the first connection 16.

Advantageously, a switch board 19 may be coupled to the control panel 15.

The control panel 15 may also be configured to control each level sensor 5, preferably via a second connection 17. The control panel 15 may be configured to allow a predetermined level of solution in each separated space 6. The control panel may be configured to open valve 4 until the level sensor senses the solution to the predetermined level. In addition, the control panel may also be configured to close valve 4 when the predetermined level is achieved. Alternatively, the control panel 15 may be configured to allow a predetermined volume of solution in each separated space 6. The control panel may be configured to open valve 4 until the predetermined volume of solution is distributed to the separated space. In addition, the control panel may also be configured to close valve 4 when the predetermined volume has been distributed to the separated space. The control panel 15 may be configured to control the duration of cycle of W-D, and for each step of Wetting and Drying.

The control panel 15 may be configured to open valve 8 and 10 when the duration of wetting (soaking) is achieved, preferably via a third connection 18; and start the drying cycle.

The control panel 15 according to the invention may be integrated into a computer system and thus be able to communicate with one or more external devices such as a keyboard, a pointing device, a display, or any device allowing a user to interact with the control panel 15. It should be understood that although not shown, other hardware and / or software components could be used in conjunction with the control panel 15. Thus, in one embodiment of the present invention, the control panel 15 may be coupled to a human machine interface (HMI). The HMI, can be used to allow the transmission of parameters to devices or conversely to provide the user with the data values measured or calculated by the device. Typically, the HMI is communicatively coupled with a processor and includes a user output interface and a user input interface. The user output interface may include an audio display, printer, video screen, pre-actuators, keyboard, sensors, buttons and output interface and various indicators such as visual indicators, audible indicators, and haptic indicators. The user input interface may include a keyboard, mouse, or other cursor navigation module such as a touchscreen, a touchpad, a stylus input interface, and a microphone for inputting audible signals such as user speech, data and commands that can be recognized by the processor.

In one embodiment of the present invention, the control panel 15 may be coupled to a communication interface, for example a network interface of Ethernet, FiberChannel, InfiniBand or any device allowing the device 1 to communicate with one or more other computing devices.

As it will be described after, such device allows an easy adaptation of parameters thanks to the at least one control panel 15.

The duration of wetting-drying (W-D) cycles required to be adapted to each type of specimen and to the (environmental) conditions it is expected to be subjected to. As mentioned, the current ASTM D559 (2015) procedure incorporates constant wetting and drying hours to specimen and ignores, amongst other parameters, the saturation level of the sample. Saturation level induced in the sample during the ingression and egression phenomenon plays an important role in the behavior of a studied structure and the studied conditions. Thus, selecting a duration of wetting and drying hours that provide the most representative saturation level experienced by sample in the field is essential to accurately estimate the behavior of a material in-situ, and is allowed by the device according to the invention.

In addition, the device allows the implementation of temperatures, which closely represent the field situation during the operation of W-D cycles. Thus, the huge difference in temperature between the wetting and drying phases in the current standard procedure, which is far from the real situation, is avoided.

Since the device allows controlling temperature, which contributes to more closely represent the in-situ environments, thus, the possible unrealistic changes either in structure or chemical composition of sample which may occur while oven-drying a sample at 71°C are prevented. Further, each sample is placed separately in different containers or spaces; thus, the physicochemical interactions between specimen and fluids and the microstructural modifications may be monitored accurately.

The device allows performing continuous wetting-drying (W-D) cycles, without interfering manipulation of the sample; thus, the obtained results at the end of each test is an impact of continuous ingression and egression phenomenon. Such continuity obviously minimizes the possible strength and microstructural modifications of the sample which cannot be avoided in the current procedure. Hence, more precise and reproducible results, which more accurately reflecting behavior of the material in-situ, can be expected using the device.

In a particular embodiment, which further contributes to the accuracy and reproducibility of the results obtained by using the device, the control panel is operatively connected to the input valve and output valve, and is configured to automatically realize their opening and/or closure for the filling and the exiting of the predetermined volume of the liquid in at least one first separated space (6), for each wetting and drying cycle.

The results more device-oriented, and consequently requires less human manipulation which in a way minimizes the disturbance of specimen and enhance reproducibility of the results.

At least two, 3, 4, 5, and even specimens can be tested at one time; hence, more results comparative in a short period of time can be achieved with minimum labor.

In addition, the invention meets the requirements of ASTM D559 (ASTM 2015) in regard with the specimen (in size, shape etc...), which is currently in use. In a particular embodiment, specimen is/are such as presenting a length to diameter ratio of 2.

Moreover, the device allows a versatile and easy adaptation of the testing conditions (temperature, wetting, and drying duration) of ingression and egression phenomenon for given types of specimens and the environmental and weathering conditions to be tested.

Specimens of different materials can be subjected to liquids with different chemistry by placing them separately in different containers/spaces. This gives the possibility to evaluate and compare the physicochemical interactions between any types of specimens and fluids accurately and separately.

Chemical nature of the supernatant of each specimen can be assayed specifically.

Impact of ingression and egression induced by a given fluid in a given type of specimen may be monitored continuously with minimum disturbance to the specimens.

According to another aspect, the invention relates to **a method 100 to simulate an in-situ wetting and drying conditions** on a sample (7) using the device according to the invention. As mentioned above said sample is preferably a civil engineering material, as but not only, as soils, compacted soils, soil treated with a binder, concretes, cements, bitumen or bituminous materials, timber, or any other material as (reinforcing or structural) steel.

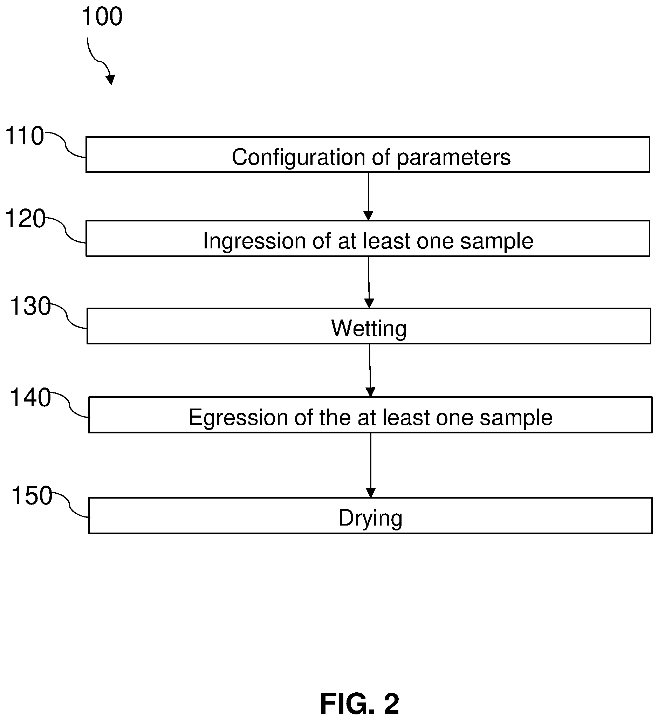

As shown in **figure 2**, in an embodiment said method 100 may comprises: a step of configuration 110 of parameters, said configuration being achieved by at least one control panel 15, a step of ingression 120 of at least one sample 7, comprising conveying the solution from a tank 2 to at least one separated space 6 comprising a sample 7, a step of wetting 130, a step of egression 140 of at least one sample 7, said egression comprising the recovery of fluid from sample 7 in at least one liquid collection tank 11, a step of drying 150.

The method 100 according to the invention comprises **a step of configuration 110** of parameters, said configuration being achieved by at least one control panel 15.

This step allows to manage parameters to reproduce features of the real conditions and environmental conditions. Several parameters may be configured as explained above.

Preferably, the step of configuration is required to be initiated by incorporating three input parameters: (i) either wetting first then drying or vice versa; (ii) duration of wetting and drying steps; (iii) the numbers of cycles the sample are to be submitted to; (iv) the cycle number after which one wish to collect the supernatant for chemical analysis.

Advantageously, input parameters comprise temperature, level of liquid.

The method 100 according to the invention comprises **a step of ingression 120** of sample 7, comprising providing a liquid and comprising conveying the liquid from a tank 2 to at least one separated space 6 comprising a sample 7. The step of ingression 120 allows to reproduce the wetting cycles and ingress which the sample is submitted to in the real conditions.

Preferably, according to the predetermined parameters, the control panel actives the pump 3. The liquid is pumped to the separated spaces containing samples through the valves 4 (as shown in Fig.1). The function, which controls the liquid pumping and the opening of valves, is designated to be controlled by the first connection 16 of the control panel 15. Once the liquid in the separated space 6 has reached the required level, the level sensor 5 is activated which is controlled by the second connection 17 of the control panel 15. The second connection 17 then triggers the first connection 16 to close the valve 4. Sample is then allowed to remain soaked as per the input wetting predetermined duration.

The method 100 according to the invention comprises **a step of egression 130** of sample 7, said egression which may comprise the recovery of fluid from sample 7 in at least one liquid collection tank 11. The step of egression allows to reproduce the egress which the sample is submitted to in the real conditions.

Preferably, on completion of the wetting predetermined duration of wetting, the egression is initiated, which includes the discharge of supernatant from the separated space, controlled by the third connection 18 of the control panel 15.

In the embodiments with at least two separated spaces, supernatants from some specimens are collected in the collection tank 9, which may either be reused or thrown out. Effluent (supernatant) of several wetting phases from at least one specimen may be gathered in at least one liquid collection tank 11 which may be later subjected to chemical analysis for example. As shown in the specific embodiment of Fig. 1, corresponding to a device with five separated spaces, supernatants from 4 specimens are collected in the collection tank 9, which may either be reused or thrown out; effluent of 4 continuous wetting phases from one specimen is gathered in 4 liquid collection tanks 11, to be later subjected to assays as chemical analysis for example.

The present scheme delineates the continuous ingress and egress operation(s) controlled by input wetting and output. As mentioned earlier, a provision will be made around the separated spaces or the climatic chamber containing specimens to regulate the temperature and RH simultaneous with the ingress and egress operation(s).

According to an embodiment, the step of ingression 120 is directly followed by the step of egression 140. In other words, the duration of the step of ingression 120 is for example between 1 min and 10000 min, preferably between 2 min and 9500min, preferably between 3 min and 9000min, preferably between 4 min and 8000min, preferably between 5 min and 7500min, preferably between 6 min and 7000min, preferably between 7 min and 6500min, preferably between 8 min and 6000min, preferably between 9min and 5500min, preferably between 10 min and 5000min, preferably between 11 min and 4500min, preferably between 12 min and 4000min, preferably between 13 min and 3500min, preferably between 14 min and 3000min, preferably between 15 min and 2500min, preferably between 16 min and 2000min, preferably between 17 min and 1500min, preferably between 18 min and 1000min, preferably between 19 min and 900min, preferably between 20 min and 800min, preferably between 21 min and 750min, preferably between 22 min and 700min, preferably between 23 min and 600min, preferably between 24 min and 500min, preferably between 25 min and 450min, preferably between 26 min and 400min, preferably between 27 min and 350min, preferably between 28 min and 300min, preferably between 29 min and 250min, preferably between 30 min and 200min, preferably between 31 min and 190min, preferably between 32 min and 180min, preferably between 33 min and 170min, preferably between 34 min and 160min, preferably between 35 min and 150min, preferably between 36 min and 140min, preferably between 37 min and 130min, preferably between 38 min and 120min, preferably between 39 min and 110min, preferably between 40 min and 100min, preferably between 41 min and 90min, preferably between 42 min and 80min, preferably between 43 min and 70min, preferably between 44 min and 60min, and more preferably between 45 min and 50min.

In this embodiment, the liquid penetrates the top of the separated space according to predetermined speed and volume, when the desired volume of solution is reached (detected by the level sensor), the solution is directly discharged thank to the valve 8,10 to the solution collection tank 9 or to the fluid collection thank(s). The inlet 4 and outlet valves 8,10 controlled by the control panel work continuously when one is open for example the inlet valve 4 the other (outlet valve 8,10) are closed and vice versa according to the predetermined inlet speed of the solution in the separate space.

According to another embodiment, the step of ingression 120 is followed by the step of egression 140 after a duration of wetting 130 (soaking). In other words, the duration of the step of ingression 120 is for example between 1 min and 10000 min, preferably between 2 min and 9500min, preferably between 3 min and 9000min, preferably between 4 min and 8000min, preferably between 5 min and 7500min, preferably between 6 min and 7000min, preferably between 7 min and 6500min, preferably between 8 min and 6000min, preferably between 9min and 5500min, preferably between 10 min and 5000min, preferably between 11 min and 4500min, preferably between 12 min and 4000min, preferably between 13 min and 3500min, preferably between 14 min and 3000min, preferably between 15 min and 2500min, preferably between 16 min and 2000min, preferably between 17 min and 1500min, preferably between 18 min and 1000min, preferably between 19 min and 900min, preferably between 20 min and 800min, preferably between 21 min and 750min, preferably between 22 min and 700min, preferably between 23 min and 600min, preferably between 24 min and 500min, preferably between 25 min and 450min, preferably between 26 min and 400min, preferably between 27 min and 350min, preferably between 28 min and 300min, preferably between 29 min and 250min, preferably between 30 min and 200min, preferably between 31 min and 190min, preferably between 32 min and 180min, preferably between 33 min and 170min, preferably between 34 min and 160min, preferably between 35 min and 150min, preferably between 36 min and 140min, preferably between 37 min and 130min, preferably between 38 min and 120min, preferably between 39 min and 110min, preferably between 40 min and 100min, preferably between 41 min and 90min, preferably between 42 min and 80min, preferably between 43 min and 70min, preferably between 44 min and 60min, and more preferably between 45 min and 50min.

In this embodiment, the solution penetrates the bottom of the separated space according to predetermined speed and volume, when the desired volume of solution is reached (detected by the level sensor), the valve 4 is closed by the control panel thank to the level sensor. The valves 8 and 10 are closed and controlled to be open after the predetermined duration. In this embodiment, the method then comprises between the step of ingression 120 and the step of egression 130 a step of soaking, preferably according to a predetermined duration. When the duration is over, the solution is discharged thank to the valve 8,10 to the solution collection tank 9 or to the fluid collection thank(s) 11. The inlet 4 and outlet valves 8,10 controlled by the control panel are closed at the same time during a predetermined period of soaking.

In this embodiment, the method may comprise a step of drying 150. A step of drying 150 may comprise the drying of the sample according to a predetermined temperature and a predetermined duration. The step of drying 150 may be before the step of ingression or after the step of egression or between the step of ingression and the step of egression. A step of drying 150 in a method is realized at a temperature inferior to 60 °C, preferably between10 °C and 60 °C, inferior to 55 °C, 50 °C, 45°C, 40 °C, 35°C, 25 °C or even 20 °C. Preferably the step of drying is performed at ambient temperature between 15 °C and 27 °C, preferably between 20-25 °C. The step of drying is realized in the separated spaces.

Advantageously, the step of ingression120 and egression 140 are performed as such to limit or avoid turbulent flow on the sample studied and therefore its possible degradation.

As mentioned above the device according to the invention make it possible to submit sample to several cycles comprising any of the sequences of steps as above without interfering manipulations of the samples. Also, in an embodiment, the method according to the invention comprises repeating 1, 2, 3, 4, 5 or even more any sequence of the steps as described above, mare particularly of the sequences of steps as described above.

Advantageously, the method may comprise a step of analyzing.

The step of analyzing may comprise the analysis of the supernatant and/or the analysis of the sample.

The analysis of the supernatant may comprise the analysis of the supernatant collected in the fluid collection thank(s) 11.

The analysis of the sample may comprise the analysis of the sample before the method and/or after the method according to the invention.

The analysis step can be implemented through sensors integrated to the device, to measure, e.g. pH of the sample, pH of the supernatant, electric conductivity and so on.

In the method of the invention, the analysis of the supernatant of a sample can be implemented at any cycle said sample is submitted to. For example, when several W-D are applied, at least one analysis step can be applied at every cycle, or only at the final cycle of the method, or, in another instance, at any predetermined cycle of the cycles applied in the method, in order to follow, e.g., the evolution of a data, or the effect of a particular parameter applied to the sample.

The features measured or assayed through said analyses may comprise: swelling of sample, chemical composition of supernatant, kinetics of the evolution of the sample or of the supernatant(s), aging kinetics of the sample, disappearance of binder, mechanical resistance (e.g. by measuring USC of the sample), composition of the remaining sample, EC, pH, impregnation, imbibition, saturation, kinetics infiltration, deformation, suction, cohesion, carbonation, ionic balance, conductimetry, pressure, erosion, size, water content, mineral concentration.

Device and method of the invention is particularly suitable for studying kinetics of evolution of said features, all through the wetting and drying protocol, without manipulating the samples or interrupting the sequences of cycles. For example, as stated above, fluids exiting from separated spaces can be analyzed all along the process as being collected in liquid collection tank(s) 11. In a possible embodiment, the fluids can be analyzed directly into the separated spaces with integrated sensor.

Thanks to the method, a controlled temperature, and ingression and egression phenomenon using different fluids close to the field situation can be reproduced. Physicochemical, mechanical, and microstructure evolution of the submitted specimens can then be monitored for several specimens at a time, separately, simultaneously, and continuously. The chemical nature of the supernatant can then be analyzed. Analyzing the evolution of these behaviors in an environment that represents a situation close to the field will definitely allow producing results that are more relevant to the in-situ sample.

A particular advantage of the method is that it allows submitting several samples (7) of different materials to the same wetting and drying conditions at the same time thereby allowing an accurate comparison of the properties of each the tested materials with minimal experimental variations. The materials (and consequently the samples) can vary in their chemical composition and/or porosity and/or compaction. This is obviously particularly cost effective and allows significant time saving.

Another particular advantage of the method is that it allows submitting several samples (7) of the same material to different wetting and drying conditions that differ by at least one parameter. This allows to accurately study the behaviour of said material to different in-situ condition with minimal experimental variation and at one time, which is also particularly cost effective and saves time. The method is particularly suitable for evaluating (the evolution of) resistance of a civil engineering material subjected to wetting and drying cycles. Said method may comprise implementing the method as described above and a step of submitting the sample of a civil engineering material to a test for determining at least one mechanical property parameter of said sample. In a particular embodiment a step of submitting the sample of a civil engineering material to a test for determining a mechanical property parameter of said sample is performed before implementing the wetting and drying cycles accordingly to the method, and after the last wetting and drying cycle.

### EXAMPLE

The device is tested for lime-treated soil, which is submitted to W-D cycles by incorporation a duration of W-D hours which represent a situation close to the field. The operation was made at room/laboratory temperature (22.1-25.2°C) and relative humidity (34.3-52.8%).

### 1. Test performed using the device.

The implementation of the device with its current ability and of the method was made on lime-treated cured specimens subjected to W-D cycles. Physicochemical characteristics and compressive strength evolution in subjected specimens were analyzed and were then compared with results obtained using the current procedure (ASTM D559).

### 2. Samples used for the test

9 Cylindrical lime-treated specimens of dimensions 0.10 m in height and 0.05 m diameter were prepared by the Static compaction method. After compaction, specimens were wrapped in plastic film and cured for 10 months at a laboratory temperature of 20 ± 1 °C.

### 3. Experimentation

The wetting and drying cycles were imposed using either the (i) ASTM D559 procedure (AP), or (ii) the method according to the invention. In the protocol according to the method according to the invention the cycles were defined to represent the ingression and egression phenomenon an in-situ soil can experience during the rainy season (defined herein as the Rainy Protocol, RP).

It was assumed that in-situ soil could reach a maximum of 85-90% saturation level and a minimum of 65-70% saturation level during rainy periods. This assumed saturation level was implemented to adapt the wetting and during hours in the present lime-treated soil. 7 hours of wetting and 17 hours of drying were found to corresponds to 85-90% and 65-70% saturation levels, respectively, when measured at the laboratory temperature of 22.1-25.2°C and Relative Humidity (RH) of 34.3-52.8%. Thus, 7 hours of wetting and 17 hours of drying were implemented at the laboratory to investigate the influence of W-D cycles on the lime-treated soil during rainy periods, and this protocol is defined as the Rainy Protocol (RP).

Of the 9 specimens prepared and cured, two sets of 4 specimens were subjected to the AP and the RP testing conditions. The remaining one was used to analyze the initial state of the soil. A total of 17 W-D cycles were operated using each of the testing conditions. The mass and volume of the specimens were recorded at the end of each cycle.

10 months cured specimen, i.e., the initial specimen, and the specimens subjected to 5th, 9th, 13th, and 17th W-D cycles in each of the two protocols were subjected to UCS test. At the end of UCS test, specimens were subjected to water content measurement by oven drying at 105°C (ASTM 2010). UCS-subjected soils were then crushed to measure the suction and pH (ASTM D4972-19 (2019)) of the specimens. The Electric Conductivity (EC) of the supernatants collected was measured. The elementary concentrations of Calcium (Ca) leached during the test in the supernatant collected were measured by subjecting it to Inductively Coupled Plasma Optical Emission Spectrometry (ICP OES) analysis.

### 4. Results - impact of AP and RP testing conditions.

### UCS evolution

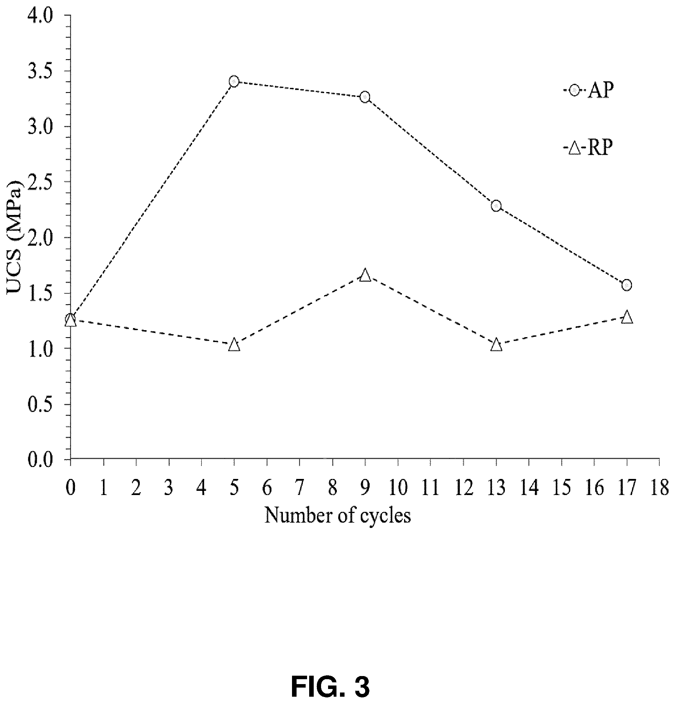

The UCS evolution of the lime-treated soil subjected to 17 alternate cycles as per the 2 protocols is presented in Fig. 3.

The UCS of the initial specimen after 10 months of curing was 1.26 MPa. This UCS increased to 3.40 MPa after the lime-treated soil goes through 5 cycles of W-D cycles as per the AP. Further increase in W-D cycles decreased the UCS to about 3.26 MPa, 2.28 MPa, and 1.57 MPa after the 9^{th}, 13^{th}, and 17^{th} W-D cycles, respectively. On the other hand, for the RP subjected soil, the overall evolution of UCS throughout the 17 W-D cycles remained almost similar compared to the initial specimen.

### Volume variations and Physicochemical evolution

The presentation of the volume changes and physicochemical properties was made in terms of percentage increase or decrease in the respective properties of the specimens with respect to the initial properties obtained from the 10 months cured specimens.

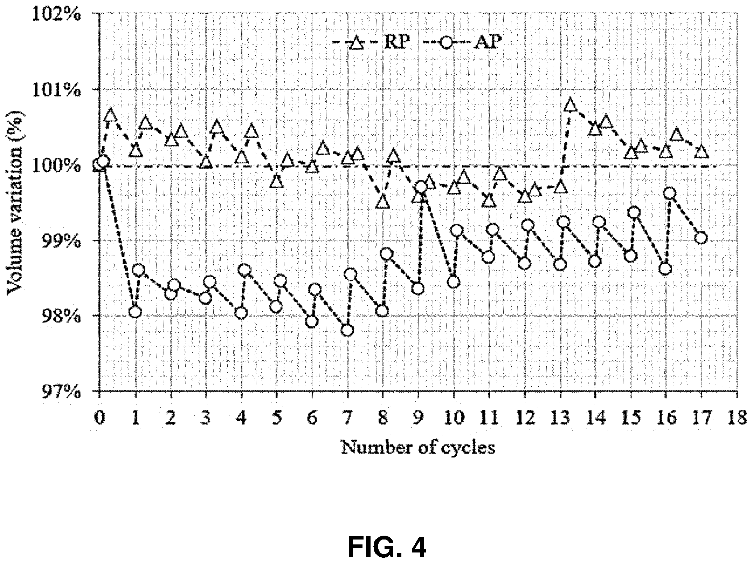

The variation in the volume of the cylindrical specimens measured after each cycle of the W-D for the AP and RPs submitted specimen is presented in Fig. 4.

In the specimens submitted to RP, the increase and decrease in the percentage of volume with respect to the initial volume between the wetting and drying phase or between the successive cycles are insignificant (Fig. 4). On the other hand, for the AP submitted specimens, a significant loss in volume of about 2% occurred compared to the initial volume of the soil. The overall volume variations were irreversible and were comparatively higher than the one experienced by the RP subjected soil.

### Water content variations

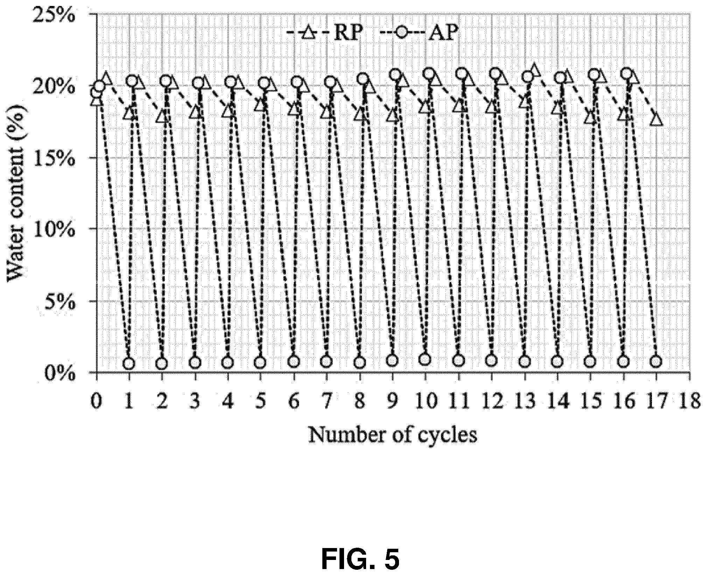

The water content was measured for the initial specimen and the UCS subjected specimens during the W-D cycles. The water content corresponding to the rest of the cycles was estimated as per the procedure provided in ASTM D 559. The evolution in water content is presented in Fig. 5.

According to Fig. 5, a maximum of 2% increase and decrease in the average water content occurred during the wetting and drying phases, respectively, at laboratory temperature in the RP-subjected soil throughout the 17 W-D cycles. This increase and decrease in the average water content were found to almost correspond with the average maximum and minimum saturation level set earlier for the RP testing condition. The global trend of average water content evolution between 2 successive wetting and drying cycles remained almost constant.

Similar to the RP-subjected soil, the overall trend in the variation of the average water content between the wetting and drying phases and between 2 successive cycles remained almost constant in the AP-subjected soil. About 1% increase in the average water content compared to the initial water content occurred during every 5 hours of wetting at laboratory temperature; however, almost a complete loss in water content occurred during each 71°C oven-drying of the AP submitted soil.

### Suction and pH evolution

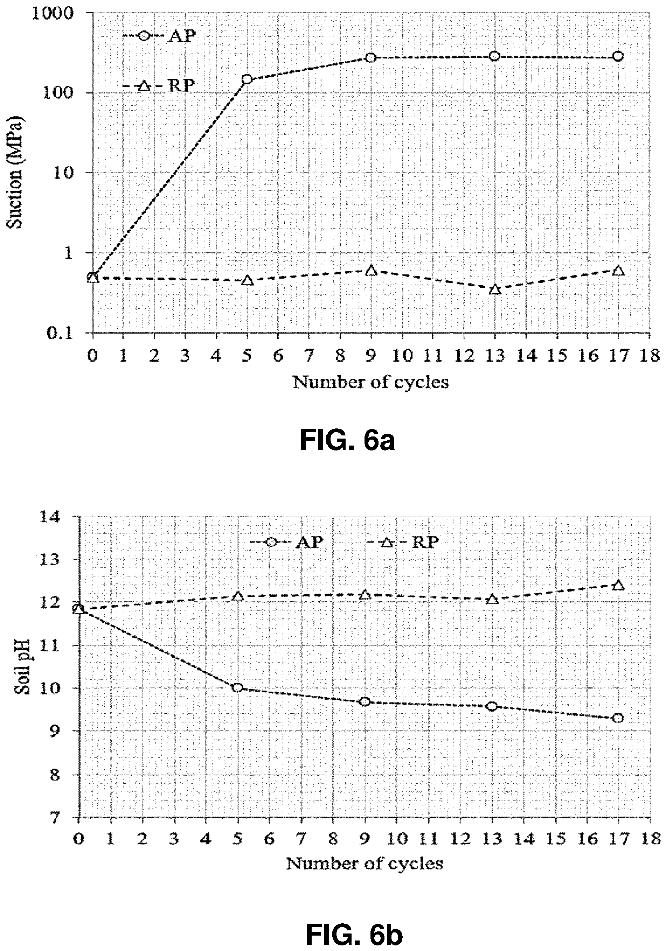

At the end of the UCS test, the soil suction and pH of the soil were measured and are presented in Fig. 6 for each protocol, with respect to the soil suction and pH recorded for the initial soil.

As expected, the soil suction of the AP submitted soil increased significantly in comparison to the initial suction of the soil (Fig. 6a). The soil suction increased to 143.5 MPa after 5^{th} cycle, then it increased to 270.0 MPa after 9^{th} cycle and then remained approximately at a similar level for the 13^{th} and 17^{th} cycles. The difference in the soil suction measured for the corresponding RP submitted soils remained less significant compared to the initial soil suction (Fig. 6a).

The soil pH measured for the initial specimen was 11.84. For the RP-subjected soil, this pH remained approximately at a similar level up to 17^{th} cycle (Fig. 6b). The pH of the AP subjected soil decreased to 10 from the initial soil pH after the 5^{th} cycle. The pH then further decreased; however, the decrease was relatively lower and remained above 9.3.

### Calcium concentration and EC evolution

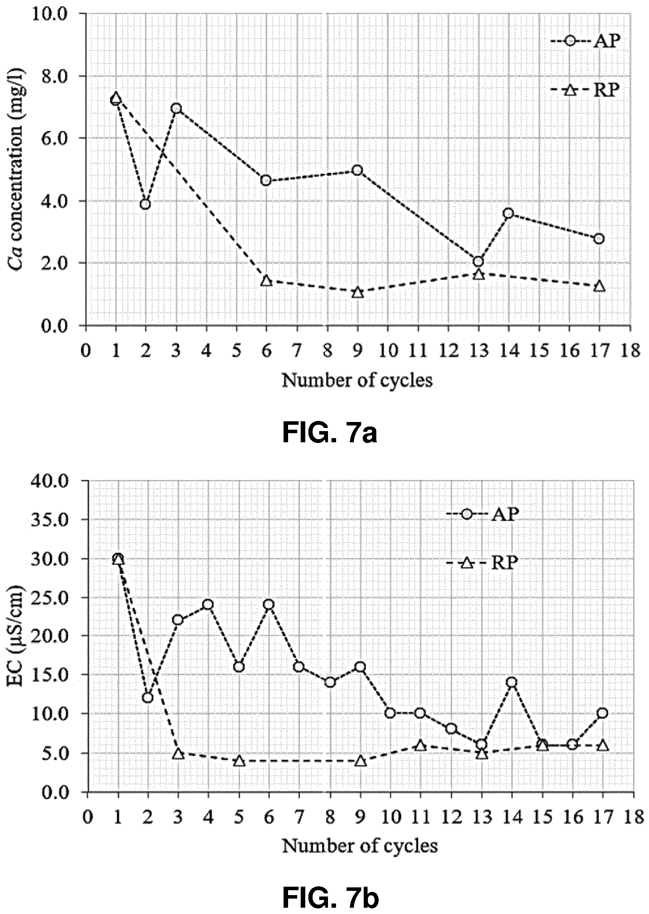

Fig. 7 presents the concentration of Calcium (Ca) (Fig 7a) and EC (Fig. 7b) of the effluents gathered during the cycles conducted as per the protocols.

The concentration of Ca leached, and the magnitude of EC remained relatively higher in AP subjected soil compared to the corresponding RP subjected soil during the W-D cycles.

The trend of the loss in Ca concentration and the measured EC decreased during the W-D cycles for the AP-subjected soil. This trend remained almost constant for the corresponding RP-subjected soil.

### 5. Conclusions

The UCS of the AP subjected soil remained higher than the initial UCS value throughout the W-D cycles (Fig. 3). On the other hand, the same configured soil, on being subjected to RP testing condition, showed a UCS evolution which is insignificant compared to the initial UCS. This observed difference makes it obvious that the UCS of a compacted and treated soil which represent the stability of a soil structure, is impacted by the implemented testing conditions. This underlines the fact that AP might lead to biased conclusions in regard with the properties of the tested materials.

In addition to an insignificant UCS evolution, the RP-subjected soil also showed an insignificant variation in volume, water content, soil suction, and pH compared to the initial soil (Fig. 4, 5, 6). On the other hand, these features changed significantly in the AP-subjected soil. A significant and irreversible volume change occurred due to total loss in water content in the AP-subjected soil during oven-drying at 71°C (Fig. 4,5). Such a loss in water content increased the matric suction of the soil, which increased the total soil suction to a value, which was much higher than the one observed in the corresponding RP-subjected soil (Fig. 6a). Thus, this makes it obvious how an elevated temperature can modify the soil structure. An induced high soil suction in the AP-subjected soil thus resulted in an overestimated UCS value. Again, the loss in soil pH in the AP-subjected soil (Fig. 6b) indicates the loss of lime components from the soil, as evident by the relative greater Calcium leaching (Fig. 7a). Besides, this loss is probably also an impact of oven drying at 71°C, as exposing a treated soil to elevated temperature was shown to negatively impact the formation of cementitious compounds.

Thus, it is obvious that implemented testing conditions can significantly modify the physicochemical behavior of soil, which can lead to a huge difference in the obtained UCS.

The above results stress the essentiality of analyzing the physicochemical evolution in a specimen before concluding the UCS observations. Such analyses are conveniently allowed with the device and method according to the invention. The current standard ASTM procedure does not provide the facility of consideration of the physicochemical interactions between specimens and fluids, as all the specimens are placed in the same cell. Thus, the conclusion regarding the obtained UCS using the current ASTM procedure may not be accurate.

The physicochemical evolution was observed to be significantly different when the ingression and egression phenomenon occurred in a laboratory temperature (using the proposed method and device) and the one that occurred between laboratory and oven-drying temperatures (as per ASTM standard). Thus, consideration of temperature should represent a situation close to the field.

The properly chosen wetting and drying durations thanks to the device of the invention allowed to make consideration of the saturation level of the given soil and points out the essential role of soil structure in UCS evolution. Since saturation level is ignored in the current standard procedure, a total loss in water occurred during drying, which modified the soil suction and gave an overestimated UCS (Fig. 3).

## Claims

1. Device (1) to simulate wetting and drying in-situ conditions on at least two samples (7) of a civil engineering material or a soil sample, for a predetermined number of wetting and drying cycles, comprising :
- a tank (2) adapted to comprise a multiple of a predetermined volume of a liquid, said predetermined volume corresponding to one cycle per said at least two samples,
- at least one input valve (4),
- at least two separated spaces (6) adapted to accommodate each sample of the at least two samples (7) and fluidically connected to the tank (2) in a controlled manner by operating the input valve (4) so that one predetermined volume of the liquid fills the separated space (6) and immerses each sample (7) at each wetting,
- at least one output valve (8, 10) for controlling the output of a supernatant which is the liquid exiting from each separated space (6) at each drying in which the sample (7) is left,
- at least one liquid collection tank (9, 11, 12, 13, 14) configured to recover the supernatant, said liquid collection tank (9, 11, 12, 13, 14) and the separated spaces (6) being fluidically connected in a controlled manner by operating the output valve (8, 10) so that the supernatant leaves the separated spaces (6),
- a climatic chamber configured to receive the separated spaces (6), which regulates the temperature and relative humidity around and/or in said separated space (6) during the wetting and drying cycle,
at least one control panel (15) configured to implement and/or monitor predetermined parameters simulating wetting and drying in-situ conditions, in said separated spaces (6), during each wetting and drying cycle, said predetermined parameters being independently implemented and/or monitored in each of the separated spaces (6) from said control panel, wherein the control panel is operatively connected to the input valve and output valve and is configured to automatically realize their opening and/or closure for the filling and the exiting of the predetermined volume of the liquid in at least one of the separated spaces (6), for each wetting and drying cycle,
wherein the separated spaces are linked at their exit
- all to one liquid collection tank (9),
- or each to at least one liquid collection tank (11, 12, 13, 14).

2. Device (1) according to the claim 1, **characterized in that** the predetermined parameters are selected from: the predetermined volume, duration of wetting, duration of drying, number of wetting and drying cycles, temperature of the liquid, temperature within the separated space, pH, electric conductivity, input flows and/or output flows, of the liquid in the at least two separated spaces (6).

3. Device (1) according to any one of preceding claims, wherein the device comprises several liquid collection tanks (11, 12, 13, 14) each configured to recover a predetermined volume of supernatant exiting from the at least two separated spaces (6), at the end of different wetting, so as to realize a given analysis of chemical/mineral species of the sample after each given wetting.

4. Device (1) according to anyone of the claims 1 to 3, **characterized in that** the device (1) comprises:
- a pump (3);
- at least one sensor to monitor at least one predetermined parameter in each separated space individually, for instance the level of liquid;
- at least one valve (4) controlling, independently from the other separated spaces, the input flow in each of the separated spaces, and/or
- at least one valve (8, 10) with controlled opening for controlling the output flow of the supernatant in one or several collection tanks.

5. Device (1) according to the claims 1-4, **characterized in that** the device (1) is configured to receive :
- the soil sample which is treated with a binder, the binder being mineral and/or organic, for instance lime, cement, fly ash, vegetable-based adhesive, resin, copolymers, or
- the civil engineering material which is soil, compacted soil, concrete, cement, bitumen or bituminous material, timber or any other material as steel.

6. Method (100) to simulate wetting and drying in-situ conditions on several samples (7) of a civil engineering material or a soil sample, comprising using the device (1) according to any one of preceding claims, said method comprising:
- a step of configurating (110) parameters, said configurating step being achieved by the at least one control panel (15);
- a step of ingression (120) of at least two samples (7), comprising conveying the liquid from a tank (2) to at least two separated spaces (6), each comprising a sample (7), the liquid immersing the at least two samples (7);
- a step of wetting (130), preferably of a predetermined period of time and/or temperature;
- a step of egression (140) of the at least two samples (7), said egression comprising the recovery of supernatant from at least two samples (7) in at least one liquid collection tank (11);
- a step of drying (150) the at least two samples (7), to obtain one wetting and drying cycle;
- and the steps of ingression, wetting, egression, drying which are repeated at least once for obtaining at least one wetting and drying cycle.

7. Method (100) according to claim 6, **characterized in that** the step of drying the samples (7) is realized at a temperature inferior to 60°C, preferably between 15 and 27°C.

8. Method according to anyone of claim 6 or 7, **characterized in that** the duration of wetting and/or drying step, is defined as to reach a predetermined liquid saturation level in the at least two samples (7).

9. Method according to any one of claims 6 to 8, **characterized in that** each sample undergoes several wetting and drying cycles.

10. Method according to any one of claims 6 to 9, **characterized in that** each of several samples (7) is submitted to at least one different parameter of wetting and drying conditions.

11. Method according to any one of claims 6 to 10, **characterized in that** it comprises a step of performing at least one physico-chemical assay on at least one supernatant exiting from the at least two separated spaces.

## Patentansprüche

1. Vorrichtung (1) zur Simulation von In-situ-Befeuchtungs- und Trocknungsbedingungen auf mindestens zwei Proben (7) eines Baustoffs oder einer Bodenprobe während einer vorbestimmten Anzahl von Befeuchtungs- und Trocknungszyklen, umfassend:
- einen Tank (2), der dazu geeignet ist, ein Vielfaches eines vorbestimmten Volumens einer Flüssigkeit zu umfassen, wobei das vorbestimmte Volumen einem Zyklus für jede der mindestens zwei Proben entspricht,
- mindestens ein Einlassventil (4),
- mindestens zwei getrennte Räume (6), die dazu geeignet sind, jede Probe der mindestens zwei Proben (7) aufzunehmen, und auf eine geregelte Weise durch Betätigen des Einlassventils (4) fluidisch mit dem Tank (2) verbunden sind, derart dass ein vorbestimmtes Volumen der Flüssigkeit den getrennten Raum (6) füllt und jede Probe (7) bei jeder Befeuchtung eintaucht,
- mindestens ein Auslassventil (8, 10) zum Regeln des Auslasses eines Überstands, der die Flüssigkeit ist, die bei jedem Trocknen aus jedem getrennten Raum (6) austritt, in dem die Probe (7) gelassen wird,
- mindestens einen Flüssigkeitssammeltank (9, 11, 12, 13, 14), der dazu ausgestaltet ist, den Überstand rückzugewinnen, wobei der Flüssigkeitssammeltank (9, 11, 12, 13, 14) und die getrennten Räume (6) auf eine geregelte Weise durch Betätigen des Auslassventils (8, 10), derart dass der Überstand die getrennten Räume (6) verlässt, fluidisch verbunden sind,
- eine Klimakammer, die dazu ausgestaltet ist, die getrennten Räume (6) aufzunehmen, und die die Temperatur und relative Feuchte um und/oder in den/dem getrennten Raum (6) während des Befeuchtungs- und Trocknungszyklus regelt, mindestens ein Bedienfeld (15), das dazu ausgestaltet ist, vorbestimmte Parameter zu implementieren und/oder zu überwachen, die In-situ-Befeuchtungs- und Trocknungsbedingungen in den getrennten Räumen (6) während jedes Befeuchtungs- und Trocknungszyklus simulieren, wobei die vorbestimmten Parameter in jedem der getrennten Räume (6) von dem Bedienfeld unabhängig implementiert und/oder überwacht werden, wobei das Bedienfeld betriebsfähig mit dem Einlassventil und Auslassventil verbunden ist und dazu ausgestaltet ist, ihre Öffnung und/oder Schließung für das Füllen und den Austritt des vorbestimmten Volumens der Flüssigkeit in mindestens einem der getrennten Räume (6) für jeden Befeuchtungs- und Trocknungszyklus automatisch auszuführen,
wobei die getrennten Räume an deren Austritt verbunden sind
- alle mit einem Flüssigkeitssammeltank (9),
- oder jeweils mit mindestens einem Flüssigkeitssammeltank (11, 12, 13, 14).

2. Vorrichtung (1) nach Anspruch 1, **dadurch gekennzeichnet, dass** die vorbestimmten Parameter ausgewählt sind aus: dem vorbestimmten Volumen, der Dauer der Befeuchtung, der Dauer der Trocknung, der Anzahl der Befeuchtungs- und Trocknungszyklen, der Temperatur der Flüssigkeit, der Temperatur innerhalb des getrennten Raums, dem pH-Wert, der elektrischen Leitfähigkeit, den Einlassströmen und/oder Auslassströmen der Flüssigkeit in den mindestens zwei getrennten Räumen (6).

3. Vorrichtung (1) nach einem der vorhergehenden Ansprüche, wobei die Vorrichtung mehrere Flüssigkeitssammeltanks (11, 12, 13, 14) umfasst, die jeweils dazu ausgestaltet sind, ein vorbestimmtes Volumen an Überstand, das von den zwei getrennten Räumen (6) am Ende einer unterschiedlichen Befeuchtung austritt, rückzugewinnen, um eine gegebene Analyse von chemischen/mineralischen Spezies der Probe nach jeder gegebenen Befeuchtung auszuführen.

4. Vorrichtung (1) nach einem der Ansprüche 1 bis 3, **dadurch gekennzeichnet, dass** die Vorrichtung (1) umfasst:
- eine Pumpe (3);
- mindestens einen Sensor zum einzelnen Überwachen mindestens eines vorbestimmten Parameters, zum Beispiel des Flüssigkeitspegels, in jedem getrennten Raum,
- mindestens ein Ventil (4), das unabhängig von den anderen getrennten Räumen die Eintrittsströmung in jedem der getrennten Räume regelt, und/oder
- mindestens ein Ventil (8, 10) mit geregelter Öffnung zum Regeln der Austrittsströmung des Überstands in einem oder mehreren Sammeltanks.

5. Vorrichtung (1) nach Anspruch 1 bis 4, **dadurch gekennzeichnet, dass** die Vorrichtung (1) ausgestaltet ist zum Aufnehmen:
- der Bodenprobe, die mit einem Bindemittel behandelt ist, wobei das Bindemittel mineralisch und/oder organisch ist, zum Beispiel Kalk, Zement, Flugasche, pflanzliches Haftmittel, Harz, Copolymere, oder
- des Baumaterials, das Boden, verdichteter Boden, Beton, Zement, Bitumen oder bituminöses Material, Holz oder ein beliebiges anderes Material wie Stahl ist.

6. Verfahren (100) zum Simulieren von In-situ-Befeuchtungs- und Trocknungsbedingungen auf verschiedenen Proben (7) eines Baumaterials oder einer Bodenprobe, das das Verwenden der Vorrichtung (1) nach einem der vorhergehenden Ansprüche umfasst, wobei das Verfahren umfasst:
- einen Schritt des Konfigurierens (110) von Parametern, wobei der Konfigurierungsschritt durch das mindestens eine Bedienfeld (15) bewerkstelligt wird;
- einen Schritt des Eintritts (120) von mindestens zwei Proben (7), der das Befördern der Flüssigkeit von einem Tank (2) zu mindestens zwei getrennten Räumen (6) umfasst, von denen jeder eine Probe (7) umfasst, wobei die Flüssigkeit die mindestens zwei Proben (7) eintaucht;
- einen Schritt des Befeuchtens (130), vorzugsweise mit einer vorbestimmten Zeit und/oder Temperatur;
- einen Schritt des Austritts (140) der mindestens zwei Proben (7), wobei der Austritt das Rückgewinnen von Überstand von mindestens zwei Proben (7) in mindestens einem Flüssigkeitssammeltank (11) umfasst;
- einen Schritt des Trocknens (150) der mindestens zwei Proben (7), um einen Befeuchtungs- und Trocknungszyklus zu erhalten;
- und die Schritte des Eintritts, Befeuchtens, Austritts, Trocknens, die mindestens einmal wiederholt werden, um mindestens einen Befeuchtungs- und Trocknungszyklus zu erhalten.

7. Verfahren (100) nach Anspruch 6, **dadurch gekennzeichnet, dass** der Schritt des Trocknens der Proben (7) bei einer Temperatur unter 60°C, vorzugsweise zwischen 15 und 27°C, durchgeführt wird.

8. Verfahren nach einem der Ansprüche 6 oder 7, **dadurch gekennzeichnet, dass** die Dauer des Befeuchtungs- und/oder Trocknungsschritts so definiert wird, dass ein vorbestimmter Flüssigkeitssättigungsgrad in den mindestens zwei Proben (7) erreicht wird.

9. Verfahren nach einem der Ansprüche 6 bis 8, **dadurch gekennzeichnet, dass** jede Probe mehrere Befeuchtungs- und Trocknungszyklen durchläuft.

10. Verfahren nach einem der Ansprüche 6 bis 9, **dadurch gekennzeichnet**, das jede von mehreren Proben (7) mindestens einem unterschiedlichen Parameter von Befeuchtungs- und Trocknungsbedingungen unterzogen wird.

11. Verfahren nach einem der Ansprüche 6 bis 10, **dadurch gekennzeichnet, dass** es einen Schritt des Durchführens mindestens einer physikalisch-chemischen Untersuchung auf mindestens einem Überstand umfasst, der aus den mindestens zwei getrennten Räumen austritt.

## Revendications

1. Dispositif (1) pour simuler des conditions de mouillage et de séchage in situ sur au moins deux échantillons (7) d'un matériau de génie civil ou d'un échantillon de sol, pour un nombre prédéterminé de cycles de mouillage et de séchage, comprenant :
- une cuve (2) adaptée pour comprendre un multiple d'un volume prédéterminé d'un liquide, ledit volume prédéterminé correspondant à un cycle pour lesdits au moins deux échantillons,
- au moins une vanne d'entrée (4),
- au moins deux espaces séparés (6) adaptés pour loger chaque échantillon parmi les au moins deux échantillons (7) et en connexion fluidique avec la cuve (2) d'une manière contrôlée par l'actionnement de la vanne d'entrée (4) de façon qu'un volume prédéterminé du liquide remplisse l'espace séparé (6) et immerge chaque échantillon (7) à chaque mouillage,
- au moins une vanne de sortie (8, 10) pour commander la sortie d'un surnageant qui est le liquide sortant de chaque espace séparé (6) à chaque séchage dans lequel l'échantillon (7) est laissé,
- au moins une cuve de collecte de liquide (9, 11, 12, 13, 14) configurée pour récupérer le surnageant, ladite cuve de collecte de liquide (9, 11, 12, 13, 14) et les espaces séparés (6) étant en connexion fluidique d'une manière contrôlée par l'actionnement de la vanne de sortie (8, 10) de façon que le surnageant quitte les espaces séparés (6),
- une enceinte climatisée configurée pour recevoir les espaces séparés (6), qui régule la température et l'humidité relative autour desdits espaces séparés (6) et/ou dans ceux-ci pendant le cycle de mouillage et de séchage,
au moins un panneau de commande (15) configuré pour appliquer et/ou surveiller des paramètres prédéterminés simulant des conditions de mouillage et de séchage in situ, dans lesdits espaces séparés (6), pendant chaque cycle de mouillage et de séchage, lesdits paramètres prédéterminés étant indépendamment appliqués et/ou surveillés dans chacun des espaces séparés (6) depuis ledit panneau de commande, lequel panneau de commande est connecté de manière fonctionnelle à la vanne d'entrée et à la vanne de sortie et est configuré pour effectuer automatiquement leur ouverture et/ou leur fermeture pour le remplissage et l'évacuation du volume prédéterminé du liquide dans au moins l'un des espaces séparés (6), pour chaque cycle de mouillage et de séchage,
dans lequel les espaces séparés sont liés au niveau de leur sortie
- en totalité à une seule cuve de collecte de liquide (9),
- ou à chacune parmi l'au moins une cuve de collecte de liquide (11, 12, 13, 14).

2. Dispositif (1) selon la revendication 1, **caractérisé en ce que** les paramètres prédéterminés sont choisis parmi : le volume prédéterminé, la durée du mouillage, la durée du séchage, le nombre de cycles de mouillage et de séchage, la température du liquide, la température à l'intérieur de l'espace séparé, le pH, la conductivité électrique, les flux d'entrée et/ou les flux de sortie, du liquide dans les au moins deux espaces séparés (6).

3. Dispositif (1) selon l'une quelconque des revendications précédentes, lequel dispositif comprend plusieurs cuves de collecte de liquide (11, 12, 13, 14) configurées chacune pour récupérer un volume prédéterminé de surnageant sortant des au moins deux espaces séparés (6), à la fin d'un mouillage différent, de façon à effectuer une analyse donnée d'espèces chimiques/minérales de l'échantillon après chaque mouillage donné.

4. Dispositif (1) selon l'une quelconque des revendications 1 à 3, **caractérisé en ce que** le dispositif (1) comprend :
- une pompe (3) ;
- au moins un capteur pour surveiller au moins un paramètre prédéterminé dans chaque espace séparé individuellement, par exemple le niveau de liquide ;
- au moins une vanne (4) commandant, indépendamment des autres espaces séparés, le flux d'entrée dans chacun des espaces séparés, et/ou
- au moins une vanne (8, 10) à ouverture commandée pour commander le flux de sortie du surnageant dans une ou plusieurs cuves de collecte.

5. Dispositif (1) selon l'une quelconque des revendications 1 à 4, **caractérisé en ce que** le dispositif (1) est configuré pour recevoir :
- l'échantillon de sol qui est traité avec un liant, le liant étant minéral et/ou organique, par exemple la chaux, un ciment, des cendres volantes, un adhésif d'origine végétale, une résine, des copolymères, ou
- le matériau de génie civil qui est un sol, un sol compacté, un béton, un ciment, un bitume ou un matériau bitumineux, un bois d'œuvre ou n'importe quel autre matériau tel qu'un acier.

6. Procédé (100) pour simuler des conditions de mouillage et de séchage in situ sur plusieurs échantillons (7) d'un matériau de génie civil ou un échantillon de sol, comprenant l'utilisation du dispositif (1) de l'une quelconque des revendications précédentes, ledit procédé comprenant :
- une étape de configuration (110) de paramètres, ladite étape de configuration étant effectuée par l'au moins un panneau de commande (15) ;
- une étape d'introduction (120) d'au moins deux échantillons (7), comprenant le convoyage du liquide depuis une cuve (2) jusqu'à au moins deux espaces séparés (6), comprenant chacun un échantillon (7), le liquide immergeant les au moins deux échantillons (7) ;
- une étape de mouillage (130), de préférence pendant une période de temps et/ou à une température prédéterminées ;
- une étape d'évacuation (140) desdits au moins deux échantillons (7), ladite évacuation comprenant la récupération de surnageant à partir des au moins deux échantillons (7) dans au moins une cuve de collecte de liquide (11) ;
- une étape de séchage (150) des au moins deux échantillons (7), pour que soit obtenu un cycle de mouillage et de séchage ;
- et les étapes d'introduction, de mouillage, d'évacuation, de séchage qui sont répétées au moins une fois pour l'obtention d'au moins un cycle de mouillage et de séchage.

7. Procédé (100) selon la revendication 6, **caractérisé en ce que** l'étape de séchage des échantillons (7) est effectuée à une température inférieure à 60°C, de préférence comprise entre 15 et 27°C.

8. Procédé selon l'une quelconque des revendications 6 et 7, **caractérisé en ce que** la durée de l'étape de mouillage et/ou de séchage est définie de façon que soit atteint un niveau de saturation de liquide prédéterminé dans les au moins deux échantillons (7).

9. Procédé selon l'une quelconque des revendications 6 à 8, **caractérisé en ce que** chaque échantillon subit plusieurs cycles de mouillage et de séchage.

10. Procédé selon l'une quelconque des revendications 6 à 9, **caractérisé en ce que** chacun des plusieurs échantillons (7) est soumis à au moins un paramètre différent de conditions de mouillage et de séchage.

11. Procédé selon l'une quelconque des revendications 6 à 10, **caractérisé en ce qu'**il comprend une étape de réalisation d'au moins un dosage physico-chimique sur au moins un surnageant sortant des au moins deux espaces séparés.
